# EUROPÄISCHE PATENTSCHRIFT

(11) **EP 3 393 376 B1**
(45) Veröffentlichungstag und Bekanntmachung des Hinweises auf die Patenterteilung: **22.05.2024**
(21) Anmeldenummer: 16819516.2
(22) Anmeldetag: 16.12.2016
(51) Int. Cl.: A61B 17/17, A61B 17/80, A61B 34/10, A61B 17/00

(54) **IMPLANTAT ZUR KNOCHENVERSTÄRKUNG MIT BOHRVEKTORVORGABELOCH UND UMGRIFFSPLATTE FÜR KIEFERERSATZ SOWIE IMPLANTATHERSTELLVERFAHREN**
IMPLANT FOR REINFORCING A BONE, COMPRISING A BORE VECTOR SPECIFYING HOLE AND SURROUNDING PLATE FOR A JAW REPLACEMENT, AND IMPLANT PRODUCTION METHOD
IMPLANT POUR UNE CONSOLIDATION OSSEUSE AVEC TROU PRÉDÉFINI DE VECTEUR DE PERÇAGE ET PLAQUE D'ADHÉSION POUR REMPLACEMENT MAXILLAIRE AINSI QUE PROCÉDÉ DE FABRICATION D'UN IMPLANT

(30) Priorität: 23.12.2015 DE 102015122793
(43) Veröffentlichungstag der Anmeldung: 31.10.2018
(73) Patentinhaber: Karl Leibinger Medizintechnik GmbH & Co. KG, 78570 Mühlheim/Donau (DE)
(72) Erfinder: WAIZENEGGER, Axel, 78570 Mühlheim (DE); REINAUER, Frank, 78570 Mühlheim (DE); GELLRICH, Nils-Claudius, 78570 Mühlheim (DE); RANA, Majeed, 78570 Mühlheim (DE)
(74) Vertreter: Winter, Brandl - Partnerschaft mbB
(86) Internationale Anmeldenummer: PCT/EP2016/081535
(87) Internationale Veröffentlichungsnummer: WO 2017/108627

(56) Entgegenhaltungen:
- WO-A1-2011/136775
- WO-A1-2014/188036
- WO-A1-2015/155296
- DE-A1-102008 058 305
- US-A1- 2009 143 825
- US-A1- 2014 074 438
- US-A1- 2015 209 093

## Beschreibung

Die vorliegende Erfindung betrifft ein Implantat zum Anbringen an einem Knochen, etwa eines Säugetiers, wie eines Primaten, bspw. eines Menschen, welches zum Anliegen an der Oberfläche eines Knochens zueinander schräg stehende Knochenanlageflächen oder Knochenformschlussabschnitte aufweist, und ein Verfahren zum Herstellen eines Implantats.

Aus dem Stand der Technik sind Implantate für die Trauma- und Rekonstruktionschirurgie bekannt, welche in Form von maßgefertigten Platten ausgebildet sind. Diese werden anhand von Computertomografie-Daten (CT-Daten) patientenspezifisch angefertigt. So kann das Design dieser Platte an die individuellen anatomischen Gegebenheiten des Patienten angepasst werden.

So offenbart bspw. die DE 10 2013 102 178 A1 ein Sternum-Osteosynthese-System in Form einer Klammer, mit einem ersten Schenkel, einem zweiten Schenkel und einem Verbindungsbereich auf.

Die DE 10 2008 051 532 A1 offenbart ein Verfahren zur präoperativen Anpassung eines zur Fixierung der Bruchfragmente eines Knochens dienenden Implantats an die Außenkontur des Knochen. Hierbei wir ein digitales Modell des Implantats in einem ungebogenen Zustand zusammen mit einer digitalisierten, den Knochen mit reponierten Bruchfragmenten zeigenden Röntgenaufnahme an einem Bildschirm dargestellt und virtuell gebogen, um es an die Außenkontur des dargestellten Knochens anzupassen. Das so angepasste virtuelle Modell dient dann als Schablone für die Biegung des Implantats. US 2014/074438 A1 offenbart ein weiteres Implantat sowie ein Verfahren zur präoperativen Anpassung und ein Herstellungsverfahren eines zur Fixierung der Bruchfragmente eines Knochens dienenden Implantats an die Außenkontur des Knochen.

Die US 2014/0128923 A1 offenbart ein System, inklusive Verfahren, Vorrichtung und Ausstattung zur Fixierung von Knochen mit Knochenplatten. Ein ähnliches Implantat ist aus der US 2015/0209093 A1 bekannt.

Die US 4 364 382 A offenbart eine Fixierungsvorrichtung für Knochenfrakturen, die ohne Schrauben auskommt und durch ein Umbiegen und Eindringen in den Knochen in ihrer Position festgelegt wird und so die Frakturstelle fixiert.

Ferner ist aus der DE 299 09 616 U1 ein Beispiel für eine herkömmliche Bohrvorrichtung bekannt.

Ein solches Implantat ist plattenförmig ausgebildet, wobei die Platte an einer Knochenoberfläche anliegt. Zur Befestigung des Implantats am Knochen sind mehrere Durchgangsbohrungen auf der Platte vorgesehen, welche in ihrer (Raum-)Position und ihrer Abwinkelung / Geometrie beim operativen Einsatz, also während der Operation, individuell festgelegt werden können. So kann die Schraubenlochanordnung gemäß den individuellen anatomischen Strukturen, wie bspw. Zahnwurzeln oder Nerven, oder gemäß bereits vorhandenen oder geplanten Implantaten bestimmt werden. Eine solche individuelle Auslegung ist auch vor Ort bisher nötig, um Schäden am Patienten, wie etwa eine Beschädigung von Nerven zu vermeiden.

Jedoch ist die Schraubenposition während des operativen Einsatzes nur in einer Raumebene in einem Winkelbereich von 0° bis maximal 15° variierbar, wobei diese Raumebene senkrecht zur Plattenoberfläche ausgerichtet ist. Dadurch ist die Auswahl der Schraubenpositionen zur Befestigung des Implantats am Knochen äußerst eingeschränkt. Des Weiteren sind die bisherigen Implantate meist balkenartig ausgebildet, das bedeutet, dass das Implantat im Vergleich zu seinem Querschnitt eine große Länge aufweist. Die Breite / Höhe des Implantats beschränkt hierbei ebenfalls die Positionierung der Schrauben. Auch sind solche konventionellen Implantate ästhetisch unattraktiv und haben gewisse medizinische Nachteile.

Darüber hinaus ist bisher zum Anbringen dieses Implantats am Knochen eine Bohrschablone mit auf jene in der herkömmlichen Platte enthaltenen Plattenlöcher ausgerichteten Bohrbüchsen notwendig, um die Bohrungen im Knochen entsprechend der Planung einbringen zu können.

Durch das Anlegen der Platte an eine Knochenoberfläche entsteht eine quasi oder nahezu parallele Ausrichtung des Implantats entlang des Knochens. Da das Implantat in Form einer Platte ausgebildet ist, können die Bohrlöcher zum Einbringen der Schrauben jedoch ausschließlich auf der Frontfläche (Fläche parallel zur Knochenanlagefläche) ausgebildet werden. Dadurch ist die Positionierung der Schrauben darauf beschränkt, dass sie ausschließlich von vorne, das bedeutet, eben dieser Frontfläche aus vorgesehen werden können. Das schränkt die Einbringbarkeit einerseits, aber auch die Güte der Verbindung andererseits ein.

Aufgabe der Erfindung ist es nun, die Nachteile aus dem Stand der Technik zu vermeiden oder wenigstens zu mildern.

Diese Aufgabe wird bei einer gattungsgemäßen Vorrichtung erfindungsgemäß dadurch gelöst, dass in einem Teilbereich des in seiner Ursprungsform eingesetzten Implantats zwei Knochenformschlussabschnitte so zueinander ausgerichtet sind, dass dort ein Umgreifen des Knochens die Position des Implantats am Knochen anliegend in allen drei Raumrichtungen eindeutig, d.h. relativ zum Knochen und im Raum, (zwangs-)festlegt.

Vorteilhafte Ausführungsformen sind in den Unteransprüchen beansprucht und werden nachfolgend erläutert.

So ist es von Vorteil, wenn das Implantat so ausgerichtete Schraubenlöcher aufweist, dass diese als Bohrschablone zum Einbringen von Bohrungen in den Knochen vorgesehen / nutzbar sind. Somit dient das Implantat gleichzeitig als Bohrschablone, weshalb der Operateur keine separate Bohrschablone mehr positionieren muss. Dadurch wird die Durchführung der Bohrungen im Knochen, welche zur Aufnahme von bspw. Schrauben dienen, erheblich erleichtert. Eine schonendere Patientenbehandlung bei sogar geringerem Zeitaufwand wird möglich.

Hierbei ist es vorteilhaft, wenn die Schraubenlöcher zumindest schräg / quer oder windschief zueinander und oder eine Längsachse des Implantats ausgerichtet sind. Eine schräge Ausrichtung der Schraubenlöcher bedeutet hierbei, dass die Schrauben in einer Raumrichtung nicht parallel zueinander angeordnet sind, und windschief beschreibt die nicht parallele Ausrichtung der Schraubenlöcher in mindestens zwei Raumrichtungen. Dadurch können die Schrauben zum Befestigen des Implantats am Knochen individuell an den jeweiligen Patienten angepasst werden, und so vorgesehen werden, dass weder Nerven noch Zähne / Zahnwurzeln beschädigt werden.

Eine vorteilhafte Ausführungsform sieht vor, dass der Innendurchmesser des Schraubenlochs auf den Außendurchmesser des Bohrers und/oder des geplanten Lochs im Knochen abgestimmt ist, etwa spielfrei oder zwangsfluchtend. Dadurch dient das Implantat gleichzeitig auch als Bohrschablone.

Hierbei ist es vorteilhaft, wenn der Innendurchmesser des Schraubenlochs ca. das 0,8-, 0,85- oder 0,9- bis 0,99-fache des geplanten Knochenlochs beträgt. In diesem Bereich ist eine besonders präzise Positionierung des Knochenlochs über das im Implantat vorhandene Schraubenloch möglich.

Eine weitere vorteilhafte Ausführungsform sieht vor, dass das Schraubenloch im Bereich einer Trägerstruktur des Implantats angeordnet ist.

Hierbei ist es von Vorteil, wenn die Schraubenlöcher zur Oberfläche der Trägerstruktur geneigt / schräg ausgebildet sind. Somit ist die individuelle Positionierung der Schrauben gemäß den jeweiligen Patientendaten möglich, und gleichzeitig kann eine kraftflussoptimierte Positionierung der Schraubenlöcher vorgesehen werden.

Des Weiteren ist es von Vorteil, wenn das Implantat selbst als Bohrschablone mit Bohrbuchsen / Bohrbüchsen ausgebildet bzw. nutzbar ist. Somit kann auf die Verwendung einer separaten Bohrschablone verzichtet werden, wodurch das korrekte Positionieren und Bohren der Knochenlöcher während des operativen Eingriffs für den Operateur erleichtert wird.

Eine weitere vorteilhafte Ausführungsform sieht vor, dass die Trägerstruktur eine solche Außenkontur, etwa durch Verlängerungen, Erhabenheiten und/oder Vertiefungen aufweist, die zu sichtbaren plastischen Veränderungen an der das Implantat implantierten Person führt. Dadurch können plastische Korrekturen, bzw. eine Rekonstruktion von ursprünglich vorhandenen Konturen gleichzeitig mit dem Setzen des Implantats durchgeführt werden.

Ferner ist ein Verfahren zum Herstellen eines solchen Implantats Teil der Erfindung.

Für das Verfahren zum Herstellen eines solchen Implantats ist es von Vorteil, wenn basierend auf vorher erhaltenen patientenspezifischen Daten die Schraubenaufnahmelöcher in einen Implantatgrundkörper so eingebracht werden, dass nach der Implantierung des Implantats die Schraubenaufnahmelöcher als Zwangsführung für einen Bohrer, der zum Einbringen von Löchern in den Knochen nutzbar ist, genutzt werden.

Besonders hilfreich sind Abwandlungen, die wie folgt ausgebildet sind:
Eine mögliche Abwandlung sieht ein Implantat zum Anbringen an einen Knochen vor, mit einer Trägerstruktur, die wenigstens einen knochenaußenstrukturfolgenden Befestigungsabschnitt zum angebracht werden an den Knochen aufweist.

Hierbei ist von Vorteil, wenn ein Sockel als eine sich von der umgebenden Außenkontur der Trägerstruktur abhebende Erhöhung oder Erhabenheit ausgebildet ist, etwa nach Art eines Vorsprungs. Somit können weitere Komponenten an oder in oder über diesen Sockel schnell und dauerfest in einer vorbestimmten Position befestigt werden.

Darüber hinaus ist es von Vorteil, wenn der Sockel ein integraler, einstückiger und vorzugsweise einmaterialiger Bestandteil der Trägerstruktur ist. Durch die integrale Ausbildung von Trägerstruktur und Sockel wird eine Verbindungsstelle und somit eine potentielle Schwachstelle vermieden. Ein besonders stabiles Implantat ist die Folge.

Ein weiterer Aspekt sieht vor, dass der Sockel zum kraft-, form- und/oder stoffschlüssigen Aufnehmen der Prothese oder eines Zwischenstücks vorbereitet ist. Dadurch wird eine einfache Anbindung bzw. Aufnahme der Prothese oder des Zwischenstücks ermöglicht.

Hierbei sieht eine vorteilhafte Ausführungsform vor, dass die Vorbereitung ein Gewinde, wie ein Innen- oder Außengewinde, oder eine Retentionsform ist, also eine solche Kontur, die ein form- und / oder kraftschlüssiges Befestigen erleichtert oder ermöglicht. Die Retentionsform enthält hierbei vorteilhafterweise einen Hinterschnitt.

Darüber hinaus ist es von Vorteil, wenn die Retentionsform ein kalottenförmiges, kugelförmiges oder sphärisches Distalstück aufweist. Das Distalstück ermöglicht die einfache Anbindung an bzw. das einfache Verbinden mit der Prothese oder dem Zwischenstück.

Des Weiteren ist es von Vorteil, wenn der Sockel eine schnappverschlussartige Ausprägung besitzt. Dadurch kann die Prothese oder das Zwischenstück einfach an den Sockel angeklipst werden und auf weitere Verbindungselemente, wie bspw. Schrauben, verzichtet werden.

Eine weitere vorteilhafte Ausführungsform sieht vor, dass das Zwischenstück als ein Zahnimplantat ausgestaltet ist und vorzugsweise unter Zwischenschaltung eines Abutments einen Kunstzahn oder eine Krone hält oder zum Halten vorbereitet ist, oder als ein Abutment ausgeformt ist.

Dabei ist es von Vorteil, dass sich der Sockel entlang einer Richtung quer oder schräg zu einer Längserstreckungsrichtung der Trägerstruktur erstreckt. Das ermöglicht, dass die Neigung des Sockels der Neigung einer daran befestigten Kunstzahns / Krone entspricht. Somit ist eine Anpassung des künstlichen Zahnersatzes an die individuelle Gebissstruktur des Patienten - und somit auch eine kraftflussoptimierte Positionierung der Prothese-Implantat-Kombination - möglich.

Darüber hinaus ist es vorteilhaft, wenn mehrere Sockel vorhanden sind, die nach Art von Pfosten ausgebildet sind. Ein solcher Aufbau ermöglicht die Aufnahme mehrerer Kunstzähne und / oder Kronen oder balkenartig geformter Zwischenstücke, welche als Querbalken auf den Sockeln aufliegend alle Sockel miteinander verbinden, um die Aufnahme bzw. Anbindung der Prothese an das Implantat zu verbessern und / oder die Festigkeit der Anbindungs- bzw. Verbindungsstelle zwischen Prothese und Implantat zu erhöhen.

Eine weitere mögliche vorteilhafte Ausführungsform sieht vor, dass alle Längsachsen der Sockel quer oder schräg zur Längserstreckungsrichtung der Trägerstruktur verlaufen. Dadurch ist es möglich, jeden der Sockel für jeden Patienten individuell an die optimale Positionierung der Prothese angepasst auszurichten.

So ist es auch von Vorteil, wenn alle Längsachsen der Sockel exakt in die gleiche Raumrichtung weisen. So können die Sockel bspw. einfacher mit einem Querbalken miteinander verbunden werden, um so die Auflagefläche der Prothese zu vergrößern und / oder die Stabilität des Sitzes der Prothese verbessern.

Ein weiterer möglicher Aspekt sieht vor, dass die Trägerstruktur gitterartig ausgebildet ist oder einen oder mehrere Gitterabschnitt/e und / oder perforierte Stege aufweist. So kann zum einen Material, und somit auch Kosten eingespart werden und gleichzeitig das Einwachsen von Knochen- und/oder Weichteilgewebe in die Gitterstruktur gefördert werden, was zu einer stabilen Verbindung durch die Ausbildung einer tertiären Stabilität zwischen dem Implantat und dem das Implantat umgebenden Knochen führt.

Hierbei ist es von Vorteil, dass die Trägerstruktur, der Gitterabschnitt und / oder der Steg eine Perforation oder mehrere Perforationen nach Art eines Durchgangsloches, etwa als Bohrung aufweist. So kann die Gitterstruktur gleichzeitig auch als Befestigungsvorrichtung genutzt werden und somit auf separat vorgesehene Befestigungspunkte / -vorrichtungen verzichtet werden.

So ist es von Vorteil, dass das Durchgangsloch zur Aufnahme einer in den Knochen einzuschraubenden Schraube ausgelegt ist. Dadurch kann auf das Anbringen separater Durchgangslöcher an dem Implantat zur Aufnahme von Schrauben, verzichtet werden.

Darüber hinaus ist das Trennen oder das Beabstanden des Distalstücks von einem Zylinderstumpfabschnitt über einen Verdünnungsbereich von Vorteil, da so bereits sehr geringe Höhen zwischen Zahnimplantat und darauf sitzender Prothese realisiert werden können, weil keine Mindestlängen, wie z.B. eine Mindestgewindetiefe, beachtet werden müssen.

Es ist auch von Vorteil, wenn der Sockel eine zylindrische Außenkontur oder eine kraftflussoptimierte Außenkontur aufweist. Dadurch können Ermüdungserscheinungen des Implantatmaterials aufgrund von nicht kraftflussoptimierter Auslegung des Sockels vermieden werden.

Eine vorteilhafte Ausführungsform sieht vor, dass der Sockel eine zumindest teilweise, vorzugsweise distalseitig, hohlzylinderartige Ausprägung besitzt. Diese Form bietet die größtmögliche Variation für die Ausgestaltung der Anbindung der Prothese.

Der oder die Sockel wird / werden vorteilhafterweise knochenmaterialersetzend eingesetzt und / oder positioniert, bzw. ist / sind knochenmaterialersetzend positionierbar. Dadurch können aufwendige Knochenrekonstruktionen durch eigenes oder fremdes Knochenmaterial vermieden werden.

Ein anderer Aspekt sieht vor, dass an einem Implantat mehrere Knochenformschlussabschnitte vorhanden sind, und geometrisch so ausgebildet und so ausgerichtet sind, dass eine formschlusseingehende Anlage am Knochen, insbesondere beim Einsetzen oder im eingesetzten Zustand in den tierischen oder menschlichen Körper erzwungen ist. Dadurch kann das Implantat ohne größeren Aufwand eineindeutig platziert werden und auf separate Positionierungshilfen verzichtet werden.

Hierbei ist es von Vorteil, wenn die Knochenformschlussabschnitte so geometrisch ausgebildet und ausgerichtet sind, dass die Anlage eine einzige stabile Lagerposition des Implantats am Knochen erzwingt. Somit wird die Positionierung vereinfacht und das Risiko einer falschen Positionierung des Implantats deutlich reduziert bzw. fast gänzlich vermieden.

Ein Implantat, an dem zumindest drei räumlich voneinander getrennte Knochenformschlussabschnitte vorhanden sind hat sich als vorteilhaft gezeigt. Durch mehrere räumlich voneinander getrennte Knochenformschlussabschnitte wird die Anlage- und Positionierungsgenauigkeit des Implantats erhöht.

Des Weiteren ist es von Vorteil, wenn jeder Knochenformschlussabschnitt in einer anderen Raumrichtung an einem anderen Knochenabschnitt zum Inanlagegelangen vorbereitet ist. Dadurch wird die Anlage- und Positionierungsgenauigkeit des Implantats weiter erhöht, sowie die Positionsstabilität des Implantats verbessert. Das bedeutet, dass das Implantat in seiner Position weniger verrutschen kann.

Darüber hinaus ist die Vorbereitung des Knochenformschlussabschnitts zum Eingehen eines Umgreifens eines Knochenabschnitts vorteilhaft. Durch das Umgreifen eines Knochenabschnitts wird das Risiko eines Verrutschens des Implantats weiter reduziert.

Eine vorteilhafte Ausführungsform sieht vor, dass der Knochenformschlussabschnitt durch die Trägerstruktur oder ein davon separates Bauteil ausgebildet ist, vorzugsweise einstückig, integral und / oder einmaterialig. Durch die einteilige Ausbildung des Knochenformschlussabschnitts und der Trägerstruktur kann die Teilanzahl reduziert werden und Materialkosten eingespart werden. Darüber hinaus wird durch die einstückige Ausbildung auch die Positionierungsgenauigkeit der Trägerstruktur und / oder des separaten Bauteils erhöht.

Die patientenspezifische konfektionierte Ausbildung des Knochenformschlussabschnitts und / oder der Trägerstruktur als massives Bauteil, etwa als Stange und / oder mit an einen individuellen Knochen und CAD-/CAM-Einsatz bzgl. dieser knochennahen oder knochenbenachbarten Außenkontur, hat sich als vorteilhaft erwiesen. Dadurch ist ein für jeden Patienten individuell auf dessen Bedürfnisse abgestimmtes Implantat herstellbar.

Des Weiteren ist es vorteilhaft, dass im Knochenformschlussabschnitt zumindest ein Schraubenaufnahmeloch oder mehrere Schraubenaufnahmelöcher vorhanden sind. Somit dienen die Knochenformschlussabschnitte gleichzeitig als Bohrschablone und als Befestigungsvorrichtung zum Befestigen des Implantats am Knochen.

Eine weitere vorteilhafte Ausführungsform ist, dass der Knochenformschlussabschnitt und / oder die Trägerstruktur einen Koppelbereich oder mehrere Koppelbereiche aufweisen, um den Knochenformschlussabschnitt an der Trägerstruktur festzulegen.

Darüber hinaus hat sich das Vorhandensein von multiplen Gitterbefestigungsstellen als vorteilhaft erwiesen. So kann die Befestigung des Implantats am Knochen individuell auf den Patienten abgestimmt werden, und Nervenbahnen, sowie eventuell vorhandene Bezahnung bei der Befestigung gemieden werden.

Ferner ist ein Implantat von Vorteil, bei dem an der Trägerstruktur Befestigungsbereiche vordefiniert und geometrisch vorbereitet sind, um eine oder mehrere in den Knochen einzuschraubende Schrauben aufzunehmen, wobei davon räumlich beabstandet Sockel oder mehrere Sockel zum Aufnehmen einer Prothese vorhanden ist / sind. Somit dient die Trägerstruktur sowohl als Bohr- als auch als Positionierungsschablone. Darüber hinaus verhindert die räumliche Trennung der Befestigung des Implantats am Knochen (erste Einschraubachse) und der Befestigung der Prothese (zweite Einschraubachse) vorzeitige Ermüdungserscheinungen des Implantatmaterials aufgrund zu hoher mechanischer Belastung an einer Stelle.

Hier ist es vorteilhaft, wenn eine Längsachse durch die einzusetzende oder eingesetzte Schraube quer, schräg oder windschief zu einer Längsachse des Sockels, insbesondere zu einer Einschraubachse des Sockels, ausgerichtet ist. Dadurch kann die Richtung der eingesetzten bzw. einzusetzenden Schraube gemäß eventuell vorhandenen Einflussfaktoren, wie bspw. Nervenbahnen oder Zähne, kraftflussoptimiert gesetzt werden und darüber hinaus die bereits oben beschriebene punktuelle mechanische Überbelastung des Implantats vermieden werden.

Eine weitere vorteilhafte Ausführungsform sieht vor, dass der Befestigungsbereich um mehr als die Länge einer Schraube und / oder mehr als das 1,2-, 2- oder 3-fache der Dicke im Befestigungsbereich und weniger als das 500-fache der Länge einer Schraube und / oder weniger als das 400-fache der Dicke im Befestigungsbereich vom Sockel entfernt ist. So kann die notwendige Festigkeit des Implantats gewährleistet und vorzeitige Ermüdungserscheinungen vermieden werden.

Es hat sich als vorteilhaft gezeigt, wenn der Sockel so ausgebildet ist, dass er eine Anbindung einer Prothese oder eines Zwischenstücks nach dem Locking- oder Non-Locking-Prinzip ermöglicht.

Des Weiteren ist es von Vorteil, wenn das Implantat, etwa die Trägerstruktur und / oder der Sockel oder einer der Sockel als Reservoir für einen medizinischen Wirkstoff oder eines pharmakologischen Wirkstoffs ausgebildet ist. Dadurch ist es möglich, bspw. Wirkstoffe, insbesondere solche, die über einen längeren Zeitraum verabreicht / eingenommen werden müssen, in Form eines Drug-Release-Systems, wie bspw. eine Drug-Release-Kapsel dort zu deponieren und so zu verabreichen. Das ist besonders für Patienten, die dauerhaft medizinische oder pharmakologische Wirkstoffe einnehmen müssen, ein Vorteil, da so eine Einnahme nicht mehr vergessen und eine Überdosierung vermieden werden kann.

Ferner ist das Vorbereiten des Implantats zum Wandeln von Kauenergie und vorzugsweise zum Laden von Akkumulatoren ebenfalls vorteilhaft. Mit der so gewonnenen Energie können beispielsweise kleinere, sich im Körper befindliche Akkumulatoren mit Energie versorgt werden.

Eine weitere vorteilhafte Ausführungsform sieht vor, dass das Implantat als ein Kieferimplantat, wie ein Unter- oder Oberkieferimplantat, ausgebildet ist. Mit einem solchen Implantat können teilbezahnte, sowie zahnlose Kiefer versorgt werden.

Außerdem ist es von Vorteil, wenn die Trägerstruktur zum Ermöglichen einer teleskopierbaren Anbringung der Prothese stofflich und geometrisch ausgelegt / vorbereitet ist. Dadurch können auch Zustände mit größeren Knochendefekten, wie bspw. nach Tumoroperationen mit Entfernung von Teilen des Kiefers, behandelt werden.

So ist es auch von Vorteil, wenn die Trägerstruktur und / oder der Sockel mit einer knochenwachstumsfördernden, einer immunsystemstärkenden, einer eine antibiotische Wirkung hervorrufenden und / oder einer reservoirfunktionübernehmenden Beschichtung versehen ist, etwas unter Einsatz von Bone morphogenetic proteins (BMPs).

Darüber hinaus ist es von Vorteil, wenn ein Bestandteil oder alle Bestandteile aus Titan, einer Titanlegierung oder einer Ti-Al-Legierung aufgebaut ist / sind. Titan und Titanlegierungen besitzen eine hohe Biokompatibilität und eine hohe Inertie und sind daher als Material für ein Implantat geeignet.

Eine weitere vorteilhafte Ausführungsform sieht vor, dass auf der Trägerstruktur und / oder dem Sockel eine Positionierungshilfe vorhanden ist. Die Positionierungshilfe dient dem Operateur während des Einsetzens des Implantats zur Kontrolle der korrekten Positionierung und anschließend in einer Nachkontrolle zum Kontrollieren, ob sich das Implantat möglicherweise verschoben hat.

Hierbei ist es vorteilhaft, wenn die Positionierungshilfe als Markierung, etwa als Lasermarkierung und / oder Erhabenheit, etwa als ein Wulst, ausgebildet ist. Die Erhabenheit ist insbesondere für eine spätere Kontrolle mittels eines Röntgenbildes von Vorteil, da sie auf solchen Bildern erkennbar ist.

Die Auslegung der Trägerstruktur als Resektions-, Positionierungs- und / oder Bohrschablone ist von Vorteil. Durch die Integration dieser Funktionen in das Implantat bzw. die Trägerstruktur, kann auf zusätzliche Mittel, die üblicherweise als solche Schablonen dienen, verzichtet werden.

Des Weiteren ist es von Vorteil, wenn der Koppelbereich oder die Koppelbereiche ein sie teilweise oder vollständig durchdringendes Loch, etwas nach Art eines Bohrloches, vorzugsweise zur Aufnahme einer Schraube aufweist.

Ferner ist ein Verfahren zum Herstellen eines Implantats, mit dem Schritt des Erfassens individueller Patientendaten, etwa beinhaltend der Knochen- und / oder Weichteilkonfiguration, beinhaltend die jeweilige Außenkontur, bspw. unter Nutzung eines MRTs oder CTs, erstellen der Trägerstruktur und / oder des Sockels auf Basis der individuellen Patientendaten, bspw. mit CAD-/CAM-Techniken, vorzugsweise unter Nutzung einer Laser-Sinterfertigung, von Vorteil.

Außerdem ist ein Verfahren zum Implantieren eines wie oben beschrieben hergestellten Implantats in einen tierischen oder menschlichen Körper beschrieben.

Mit anderen Worten besteht die Erfindung darin, dass ein Implantat zur Verfügung gestellt wird, welches aufgrund von mindestens zwei Knochenformschlussabschnitten eindeutig am Knochen positionierbar ist und dessen Schraubenlöcher so ausgebildet sind, dass sie als Zwangsführung für einen Bohrer dienen, der zum Einbringen der Löcher in den Knochen verwendet wird, das bedeutet, dass das Implantat mit den Schraubenlöchern gleichzeitig als Bohrschablone dient. Des Weiteren umfasst die Erfindung ein Verfahren zum Herstellen eines solchen Implantats.

Die Erfindung wird nachfolgend mit Hilfe von Zeichnungen näher erläutert, in denen unterschiedliche Variationen dargestellt sind. Es zeigen:
- FIG. 1: eine Seitenansicht des Implantats an einem Kieferknochen in einer ersten Ausführungsform,
- FIG. 2: eine Unteransicht des am Kieferknochen verbauten Implantats aus Fig. 1,
- FIG. 3: eine Vorderansicht einer zweiten Ausführungsform eines an einem Kieferknochen verbauten Implantats,
- FIG. 4: eine Seitenansicht des Implantats aus Fig. 2,
- FIG. 5: eine Draufsicht einer dritten Ausführungsform eines an einem Kieferknochen verbauten Implantats,
- FIG. 6: eine Seitenansicht des Implantats aus Fig. 3,
- FIG. 7: eine Vorderansicht des Implantats aus Fig. 3 und 4,
- FIG. 8: eine Draufsicht einer vierten Ausführungsform eines an einem Kieferknochen verbauten Implantats,
- FIG. 9: eine Vorderansicht des Implantats aus Fig. 8, und
- FIG. 10: eine Seitenansicht des Implantats aus Fig. 8 und 9.

Die Figuren sind lediglich schematischer Natur und dienen nur dem Verständnis der Erfindung. Die gleichen Elemente sind mit denselben Bezugszeichen versehen. Merkmale der einzelnen Ausführungsformen können auch in anderen Ausführungsformen realisiert werden. Sie sind also untereinander austauschbar.

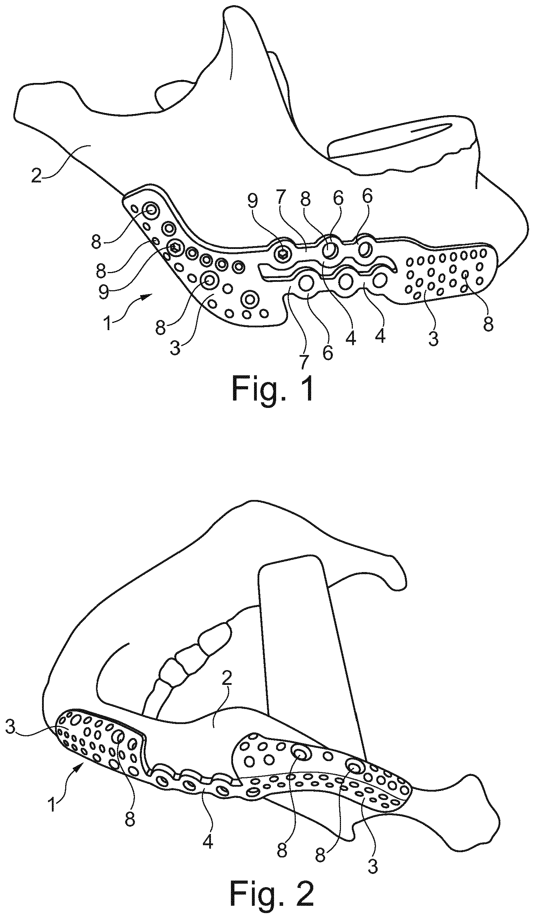

FIG. 1 zeigt eine Seitenansicht des Implantats 1 in einer ersten Ausführungsform. Das Implantat 1 dient in dieser Ausführungsform als ein Unterkieferimplantat, welches an einem Knochen 2 des Unterkiefers anliegt. Das Implantat 1 verfügt über Knochenkonturabschnitte 4, sowie Knochenanlageflächen / Knochenformschlussabschnitte 3. Die Knochenformschlussabschnitte 3 dienen einem Umgreifen des Knochens 2, wodurch die Position des Implantats 1 eindeutig festgelegt wird (siehe auch FIG. 2).

Die Knochenkonturabschnitte 4 und die Knochenformschlussabschnitte 3, wie sie in Fig. 1 dargestellt sind, bilden zusammen eine Trägerstruktur 5 des Implantats 1 aus, wobei die Knochenformschlussabschnitte 3 massiver ausgebildet sind als die Knochenkonturabschnitte 4. Die Knochenkonturabschnitte 4 sind in dieser Ausführungsform gitterartig ausgebildet, und weisen Ringabschnitte 6 auf, die über Stege 7 miteinander und mit den Knochenformschlussabschnitten 3 verbunden sind. Die Ringabschnitte 6 dienen hierbei als Schraubenaufnahmelöcher 8.

Die Knochenformschlussabschnitte 3 verfügen über mehrere Schraubenaufnahmelöcher 8 mit unterschiedlichen Innendurchmessern. An dem proximalen Knochenformschlussabschnitt 3 (links in dieser Abbildung) sowie an dem medialen Knochenkonturabschnitt 4 sind beispielhaft Schrauben 9 zu sehen, welche zur Befestigung des Implantats 1 an dem Knochen 2 dienen. Diese Schrauben 9 können bspw. als Osteosyntheseschrauben ausgebildet sein.

FIG. 2 zeigt eine Unteransicht des Implantats 1 gemäß der ersten Ausführungsform. In dieser Abbildung ist besonders gut zu erkennen, dass die Knochenformschlussabschnitte 3 den Knochen 2 bspw. flächig umgreifen, während die Knochenkonturabschnitte 4 an der Oberfläche des Knochens 2 anliegen und der Knochenkontur quasi folgen. Durch das Umgreifen des Knochens 2 des Knochenformschlussabschnittes 3 ist die Position des Implantats am Knochen 2 eindeutig festgelegt, während durch das Anlegen eines herkömmlichen Implantats, welches ausschließlich Knochenkonturabschnitte 4 aufweist, das Implantat nicht eindeutig und nachhaltig zum Knochen positioniert werden kann.

In dieser Abbildung ebenfalls gut zu erkennen sind die Schraubenlöcher 8, welche mit unterschiedlichen Innendurchmessern versehen sind. Natürlich sind auch gleiche Innendurchmesser möglich. Hierbei ist anzumerken, dass jedes Schraubenloch 8 zur Aufnahme einer Schraube 9 (siehe FIG.1) vorbereitet ist, jedoch nur ausgewählte Löcher zur Befestigung des Implantats 1 am Knochen 2 genutzt werden.

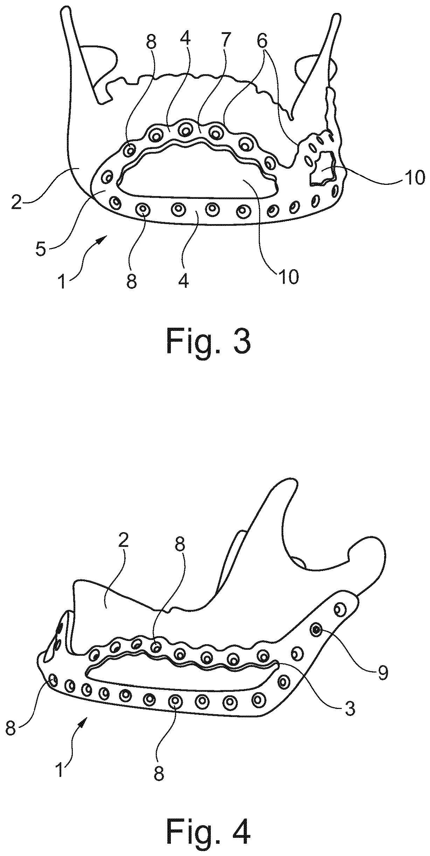

FIG. 3 zeigt eine Vorderansicht des Implantats 1 in einer zweiten Ausführungsform. In dieser Ausführungsform umfasst / umgreift das Implantat 1 den Knochen 2 eines Unterkiefers auf einer Seite (in dieser Abbildung rechts) und im Kinnbereich. In dieser Ausführungsform ist die Trägerstruktur 5 vorwiegend gitterartig, bestehend aus Ringabschnitten 6 und Stegen 7, ausgebildet. Da eine ausreichende Stabilität bereits über eine solche gitterartige Trägerstruktur 5 erreicht werden kann, können Aussparungen 10 zur Materialeinsparung vorgesehen werden.

Diese zweite Ausführungsform des Implantats 1 zeigt deutlich die knochenaußenkonturfolgende Ausbildung der Knochenkonturabschnitte 4 sowie der Knochenformschlussabschnitte 3 (siehe Fig. 4), wobei die Knochenformschlussabschnitte 3 an mehreren Bereichen eines Knochens 2 anliegen. Das bedeutet, dass ein Knochenformschlussabschnitt 3 quasi aus mindestens zwei zueinander nicht parallel ausgerichteten, miteinander verbundenen Knochenkonturabschnitte 4 ausgebildet ist. Vorzugsweise sind diese Knochenkonturabschnitten 4 einstückig und einmaterialig ausgebildet.

FIG. 4 zeigt eine Seitenansicht von lateral, d.h. in der Darstellung rechts des in FIG. 3 gezeigten Implantats 1 der zweiten Ausführungsform. In dieser Abbildung gut zu erkennen ist der Knochenformschlussabschnitt 3 am hinteren Bereich des Knochens 2 (rechts in Fig. 4). Diese Ausführungsform des Implantats 1 verfügt ebenfalls über Ringabschnitte 6, welche als Schraubenaufnahmelöcher 8 dienen und zur Aufnahme von Schrauben 9 vorbereitet sind.

Es ist zu erkennen, dass in dieser Ausführungsform des Implantats 1 die Schraubenaufnahmelöcher zueinander schräg bzw. windschief positioniert sind, und somit für den Bohrer zum Einbringen von Löchern in den Knochen 2 unterschiedliche Richtungen zwangsvorgeben. Des Weiteren sind in dieser Ausführungsform die Schraubenaufnahmelöcher 8 ebenfalls mit unterschiedlichen Innendurchmessern vorgesehen.

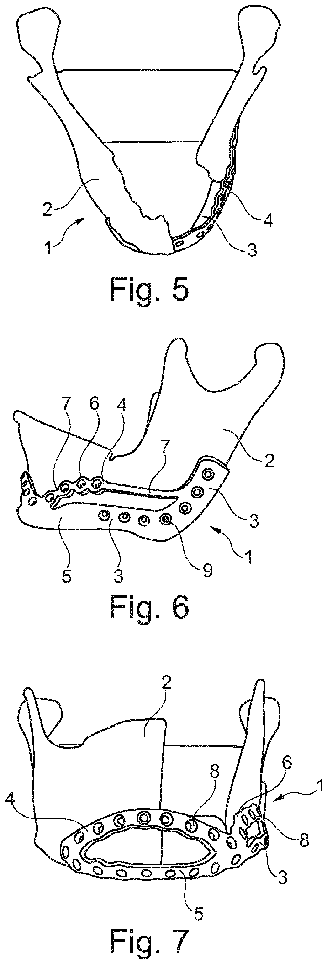

FIG. 5 zeigt eine Draufsicht des Implantats 1 in einer dritten Ausführungsform. Diese dritte Ausführungsform ähnelt der zuvor beschriebenen zweiten Ausführungsform des Implantats 1 (siehe auch FIG. 6 und FIG. 7), weist jedoch zusätzlich zu dem Knochenformschlussabschnitt 3 am hinteren Bereich des Knochens 2 auch einen Knochenformschlussabschnitt 3 am seitlichen Bereich des Knochens 2 auf.

In dieser dritten Ausführungsform dient das Implantat 1 bspw. zur Überbrückung von Kieferknochenfehlstellen und zur Stabilisierung des noch vorhandenen Kieferknochens 2. Darüber hinaus dient es im Bereich der Knochenfehlstelle als Basis für das Einbringen eines Fremdknochenteilstücks bzw. eines künstlichen Knochens (beides nicht dargestellt), um die Fehlstelle auszugleichen.

FIG. 6 zeigt eine Seitenansicht des Implantats 1 von rechts bei Orientierung an der FIG. 5. Es ist zu erkennen, dass die Trägerstruktur 5 im Bereich der Knochenformschlussabschnitte 3 massiver ausgebildet ist als im Bereich der Knochenkonturabschnitte 4, welche eine gitterartige Struktur aufweisen.

FIG. 7 zeigt eine Vorderansicht des Implantats 1 in der dritten Ausführungsform. In dieser Abbildung, sowie in FIG. 5 ist zu erkennen, dass das Implantat 1 im Bereich der Knochenfehlstelle eine individuell ausgebildete Kontur aufweist. Diese wird während der Planung anhand eines im Modell intakten Kieferknochens 2 geplant, um den später eingesetzten Fremdknochen (nicht dargestellt) präzise und exakt entsprechend der ursprünglichen Kieferknochenkontur positionieren zu können. Dadurch wird ermöglicht, dass durch den in die Fehlstelle eingebrachten Knochenersatz äußerlich keine plastischen Konturveränderungen entstehen, und auch evtl. darauf aufgebrachter Zahnersatz entsprechend der vorhandenen Gebissstruktur ausgerichtet werden kann.

Somit ist eine kraftflussoptimierte Ausrichtung bzw. Positionierung des für den Zahnersatz notwendigen Implantats möglich, wodurch die Belastung eines solchen Zahnimplantats, sowie des Implantats gemäß der Erfindung minimiert werden kann. Dadurch treten seltener oder zumindest später Ermüdungserscheinungen des Implantatmaterials auf und das Risiko, dass sich das Implantat lockert, kann deutlich reduziert werden.

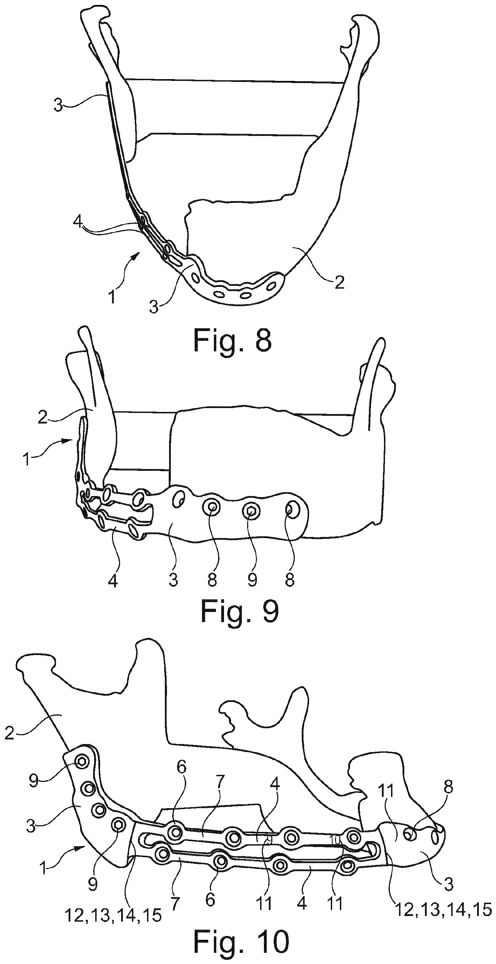

FIG. 8 zeigt eine Draufsicht des Implantats 1 in einer vierten Ausführungsform. In dieser Ausführungsform ist im Bereich der Fehlstelle des Knochens 2 kein Knochenformschlussabschnitt 3 vorgesehen, sondern lediglich Knochenkonturabschnitte 4, welche über ihre Kontur den später eingebrachten Fremdknochen exakt positionieren. Im Bereich des Kinns sowie im hinteren Bereich des Knochens 2 (siehe FIG. 10) sind Knochenformschlussabschnitte 3 vorgesehen.

Die Knochenkonturabschnitte 4 sind gitterartig ausgebildet und bestehen aus jeweils einer linienförmigen Anordnung von Ringabschnitten 6 und Stegen 7 in alternierender Weise.

FIG. 9 zeigt eine Vorderansicht des Implantats 1 in der vierten Ausführungsform. Gut zu erkennen ist hier die massivere Ausgestaltung des Knochenformschlussabschnitts 3 im Bereich des Kinns. An diesem Knochenformschlussabschnitt 3 sind mehrere Schraubenaufnahmelöcher 8 vorgesehen, die zur Aufnahme von Schrauben 9 dienen. Über die Ausrichtung eines Schraubenaufnahmelochs 8 ist der Bohrvektor für die in dem Knochen 2 einzubringenden Löcher, und somit die Position der Schraube 9 im Knochen 2, definiert und/oder zwangsvorgegeben.

FIG. 10 zeigt eine Seitenansicht des Implantats 1 in der vierten Ausführungsform. In dieser Abbildung ist zu erkennen, dass auch der Knochenformschlussabschnitt 3 am hinteren Bereich des Knochens 2 massiver ausgebildet ist als die Knochenkonturabschnitte 4, welche die beiden Knochenformschlussabschnitte 3 miteinander verbinden.

Das Implantat 1 verfügt in dieser Ausführungsform über eine Nummerierung 11 der Schraubenaufnahmelöcher 8, sowie über zwei Markierungen 12, wovon sich je eine am linken bzw. am rechten Ende der Knochenkonturabschnitte 4 befindet.

Die Nummerierung 11 der Schraubenaufnahmelöcher 8 dient dem Operateur als Orientierungshilfe, da nicht alle Schraubenaufnahmelöcher 8 zur Befestigung des Implantats 1 am Knochen 2 mittels Schrauben 9 genutzt werden. Über die Nummerierung 11 kann der Operateur während des operativen Eingriffs nachvollziehen, in welche Schraubenaufnahmelöcher 8 Schrauben 9 gesetzt werden sollen, und er kann überprüfen, ob er alle notwendigen Schrauben 9 gesetzt hat.

Die Markierungen 12 sind als Lasermarkierungen 13 und/oder Erhabenheiten 14 ausgebildet, welche mittels einer Sonde (nicht dargestellt) erkennbar sind. Die Markierungen können auch solcher Natur sein, dass sie in einer CT-Datenerfassung und/oder einem Röntgenbild erkennbar sind. Über solche Markierungen 12 kann der Operateur zum einen die korrekte Positionierung des Implantats 1 überprüfen. Zum anderen können diese Markierungen 12 dazu genutzt werden, den Bereich zu markieren, in welchem der Knochen 2 entfernt werden muss (bspw. aufgrund von vorhandenem Tumorgewebe) und dienen somit während des Eingriffs als Kontrollmarkierungen 15, die der Operateur mit Hilfe einer Sonde ertasten kann und/oder optisch / haptisch erfassen kann, und somit überprüfen kann, ob er den zu entfernenden Knochenbereich vollständig entfernt hat.

### Bezugszeichenliste

- 1: Implantat
- 2: Knochen
- 3: Knochenformschlussabschnitt
- 4: Knochenkonturabschnitt
- 5: Trägerstruktur
- 6: Ringabschnitt
- 7: Steg
- 8: Schraubenaufnahmeloch
- 9: Schraube
- 10: Aussparung
- 11: Nummerierung
- 12: Markierung
- 13: Lasermarkierung
- 14: Erhabenheit
- 15: Kontrollmarkierung

## Patentansprüche

1. Implantat (1) zum Anbringen an einem Knochen (2), welches zum Anliegen an der Oberfläche eines Knochens (2) zueinander schräg stehende Knochenformschlussabschnitte (3) aufweist, wobei in einem Teilbereich des in seiner Ursprungsform eingesetzten Implantats (1) zwei Knochenformschlussabschnitte (3) so zueinander ausgerichtet sind, dass dort ein Umgreifen des Knochens (2) die Position des Implantats (1) am Knochen anliegend in allen drei Raumrichtungen eindeutig festlegt, und wobei das Implantat (1) so ausgerichtete Schraubenaufnahmelöcher (8) aufweist, dass diese als Bohrschablone zum Einbringen von Bohrungen in den Knochen (2) nutzbar sind, **dadurch gekennzeichnet, dass** das Implantat (1) als Bohrschablone mit Bohrbuchsen ausgebildet ist.

2. Implantat (1) zum Anbringen an einem Knochen (2) gemäß Anspruch 1, **dadurch gekennzeichnet, dass** die Schraubenaufnahmelöcher (8) zumindest schräg oder windschief zueinander ausgerichtet sind.

3. Implantat (1) zum Anbringen an einem Knochen (2) gemäß einem der Ansprüche 1 oder 2, **dadurch gekennzeichnet, dass** ein Innendurchmesser eines der Schraubenaufnahmelöcher (8) auf den Außendurchmesser des Bohrers und/oder des geplanten Lochs im Knochen (2) abgestimmt ist.

4. Implantat (1) zum Anbringen an einem Knochen (2) gemäß Anspruch 3, **dadurch gekennzeichnet, dass** der Innendurchmesser des Schraubenaufnahmelochs (8) ca. das 0,8-, 0,85- oder 0,9- bis 0,99-fache des geplanten Knochenlochs beträgt.

5. Implantat (1) zum Anbringen an einem Knochen (2) gemäß einem der Ansprüche 1 bis 4, **dadurch gekennzeichnet, dass** das Schraubenaufnahmeloch (8) im Bereich einer Trägerstruktur (5) des Implantats (1) angeordnet ist.

6. Implantat (1) zum Anbringen an einem Knochen (2) gemäß Anspruch 5, **dadurch gekennzeichnet, dass** die Schraubenaufnahmelöcher (8) zur Oberfläche der Trägerstruktur (5) geneigt und/oder schräg ausgebildet sind.

7. Verfahren zum Herstellen eines Implantats (1) nach einem der vorhergehenden Ansprüche mit den folgenden Schritten:
- Erfassen der patientenspezifischen Knochenstruktur mittels MRT und/oder CT;
- Erstellen eines CAD-Modells dieser spezifischen Knochenstruktur;
- Erstellen eines CAD-Modells eines individuell ausgeprägten Implantats basierend auf dem CAD-Modell der patientenspezifischen Knochenstruktur; und
- Fertigen des individuell ausgeprägten Implantats basierend auf dem CAD-Modell.

8. Verfahren zum Herstellen eines Implantats (1) gemäß Anspruch 7 **dadurch gekennzeichnet, dass** basierend auf vorher erhaltenen patientenspezifischen Daten die Schraubenaufnahmelöcher (8) so eingebracht werden, dass nach der Implantierung des Implantats (1) die Schraubenaufnahmelöcher (8) als Zwangsführung für einen Bohrer, der zum Einbringen von Löchern in den Knochen (2) nutzbar ist, verwendbar sind.

## Claims

1. An implant (1) for attaching to a bone (2), having bone formfitting sections (3) which are inclined relative to each other to rest on the surface of a bone (2), wherein two bone formfitting sections (3) are aligned relative to each other in a sub-region of the implant (1) inserted in its original shape such that surrounding of the bone (2) definitely secures the position of the implant (1) abutting on the bone in all three spatial directions, and wherein the implant (1) includes screw seating holes (8) aligned such that they can be used as a boring template for introducing bores into the bone (2), **characterized in that** the implant (1) is in the form of a boring template comprising drill guide bushes.

2. The implant (1) for attaching to a bone (2) according to claim 1, **characterized in that** the screw seating holes (8) are aligned at least diagonally or skew relative to each other.

3. The implant (1) for attaching to a bone (2) according to any one of the claims 1 or 2, **characterized in that** an inner diameter of one of the screw seating holes (8) is adjusted to the outer diameter of the drill and/or of the intended hole in the bone (2).

4. The implant (1) for attaching to a bone (2) according to claim 3, **characterized in that** the inner diameter of the screw seating hole (8) amounts to about 0.8, 0.85 or 0.9 to 0.99 times the intended bone hole.

5. The implant (1) for attaching to a bone (2) according to any one of the claims 1 to 4, **characterized in that** the screw seating hole (8) is arranged in the area of a support structure (5) of the implant (1).

6. The implant (1) for attaching to a bone (2) according to claim 5, **characterized in that** the screw seating holes (8) are formed to be inclined and/or diagonal relative to the surface of the support structure (5).

7. A method for producing an implant (1) according to any one of the preceding claims comprising the following steps of:
- capturing the patient-specific bone structure by means of MRT and/or CT;
- creating a CAD model of said specific bone structure;
- creating a CAD model of an individually shaped implant based on the CAD model of the patient-specific bone structure; and
- producing the individually shaped implant based on the CAD model.

8. The method for producing an implant (1) according to claim 7, **characterized in that,** based on previously obtained patient-specific data, the screw seating holes (8) are introduced such that after implanting the implant (1) the screw seating holes (8) can be used as a forced guide for a drill which can be employed for introducing holes into the bone (2).

## Revendications

1. Implant (1) destiné à être appliqué à un os (2), implant qui présente des sections d'ajustement de forme osseuse (3) inclinées l'une par rapport à l'autre pour reposer au niveau de la surface d'un os (2), dans lequel, dans une zone partielle de l'implant (1) utlisé dans sa forme originale, deux sections d'ajustement de forme osseuse (3) sont alignées les unes par rapport aux autres de sorte qu'un entourage de l'os (2) détermine clairement la position de l'implant (1) reposant contre l'os dans l'ensemble des trois directions spatiales et dans lequel l'implant (1) présente des trous de réception de vis (8) alignés de sorte qu'ils puissent être utilisés en tant que gabarit de perçage pour l'introduction de trous dans l'os (2), **caractérisé en ce que** l'implant (1) est conçu en tant que gabarit de perçage avec des douilles de perçage.

2. Implant (1) destiné à être appliqué à un os (2) selon la revendication 1, **caractérisé en ce que** les trous de réception de vis (8) sont alignés au moins inclinés ou obliquement les uns par rapport aux autres.

3. Implant (1) destiné à être appliqué à un os (2) selon l'une quelconque des revendications 1 ou 2, **caractérisé en ce qu'**un diamètre intérieur d'un des trous de réception de vis (8) est adapté au diamètre extérieur du foret et/ou du trou prévu dans l'os (2).

4. Implant (1) destiné à être appliqué à un os (2) selon la revendication 3, **caractérisé en ce que** le diamètre intérieur du trou de réception de vis (8) est environ 0,8, 0,85 ou 0,9 à 0,99 fois celui du trou osseux prévu.

5. Implant (1) destiné à être appliqué à un os (2) selon l'une quelconque des revendications 1 à 4, **caractérisé en ce que** le trou de réception de vis (8) est disposé dans la zone d'une structure de support (5) de l'implant (1).

6. Implant (1) destiné à être appliqué à un os (2) selon la revendication 5, **caractérisé en ce que** les trous de réception de vis (8) sont inclinés et/ou obliques par rapport à la surface de la structure de support (5).

7. Procédé de fabrication d'un implant (1) selon l'une quelconque des revendications précédentes avec les étapes suivantes :
- détection de la structure osseuse spécifique au patient par IRM et/ou tomodensitométrie ;
- création d'un modèle CAO de cette structure osseuse spécifique ;
- création d'un modèle CAO d'un implant individualisé basé sur le modèle CAO de la structure osseuse spécifique au patient ; et
- fabrication de l'implant individualisé sur la base du modèle CAO.

8. Procédé de fabrication d'un implant (1) selon la revendication 7, **caractérisé en ce que**, sur la base de données spécifiques au patient obtenues au préalable, les trous de réception de vis (8) sont introduits de sorte qu'après l'implantation de l'implant (1), les trous de réception de vis (8) puissent être utilisés comme guidage forcé pour un foret qui peut être utilisé pour l'introduction de trous dans l'os (2).
